# EUROPEAN PATENT APPLICATION

(11) **EP 2 865 804 A1**
(43) Date of publication of application: **29.04.2015**
(21) Application number: 12744139.2
(22) Date of filing: 22.06.2012
(51) Int. Cl.: D06H 5/00, A61F 13/15, B29C 63/00, B29C 65/50

(54) **METHOD AND MACHINE FOR SEALING THREE-DIMENSIONAL FABRICS, AND THUS PRODUCED GARMENTS**

(71) Applicant: Impetus Portugal - Têxteis SA, 4740-141 Apúlia (PT)
(72) Inventor: ESTEVES DE SOUSA FANGUEI, Raul Manuel, P-4700-320 Braga (PT); QUEIROGA FIGUEIREDO, Alberto, P-4740-598 Palmeira de Faro (PT)
(74) Representative: Ferreira, Maria Silvina
(86) International application number: PCT/PT2012/000022
(87) International publication number: WO 2013/191574

(57) **Abstract**

Method for sealing a three-dimensional knitted fabric (10) of an undergarment to retain body fluids, comprising the application and hot-melt adhesion along the perimeter of the garment of a first tape (20), the tape (20) being face-to-face folded along the garment's thickness; and the application and hot-melt adhesion along the garment seams of a second tape; said tapes (20) comprising hot-melt material. The hot-melt material can be polyurethane.

The garments thus obtained may be male or female underwear for urinary incontinence.

The machine for obtaining said sealing comprises a folding element so that the tape (20) receives, within a "U"-shaped profile concavity, the garment's three-dimensional knitted fabric (10).

## Description

### Technical domain of the invention

The technology herein presented refers to the development of a process of applying a structure based on fibrous materials, which has the ability to retain fluids, particularly in incontinence garments.

### Summary of the Invention

A method is disclosed for sealing a three-dimensional knitted fabric used in male and female undergarment for urinary incontinence comprising the application along the entire absorbent structure perimeter of a side tape consisting particularly of polyurethane and polyester; and the application in the centre area, on centre seam(s), of a tape consisting particularly of polyurethane.

In one embodiment, the side tape is applied laterally in U shape, by means of an auxiliary device incorporated in the machine. In another embodiment, the side tape is fixed at a temperature between 210 and 270 °C, at an application rate between 1 and 3 m/min and at a pressure between 0.3 and 0.45 MPa. In a further embodiment, the optimum temperature is about 250 °C, the application rate is about 2.5 m/min and the pressure is of about 0.35 MPa.

In one embodiment, the centre tape is applied at a temperature between 210 and 270 °C, at an application rate between 1 and 4 m/min and at a pressure between 0.3 and 0.45 MPa. In one embodiment, the optimum temperature is of about 250 °C, the application rate is about 2.5 m/min and the pressure is about 0.35 MPa.

### Background of the Invention

Urinary incontinence occurs when the pressure inside the bladder exceeds that existing in the urethra, i.e. when there is a considerable increase in urinating pressure on the bladder, during the filling cycle of urination. Incontinence can occur at any age, but its cause tends to be different for each age group. Non-woven materials are normally used as the absorbent structure in undergarment, in particular in male underwear. Types of urinary incontinence include: stress, filling, total, psychogenic and mixed incontinence.

The products available in the market for urinary incontinence, particularly male incontinence, include sanitary napkins and diapers, underwear with pockets for a sanitary napkin, underwear with fixed sanitary napkin and underwear with embedded absorbent structure.

Products based on three-dimensional knitted fabrics are known, in particular, those described in WO2011/108954.

However, available solutions are characterized by the inability of liquid retention within those structures.

In terms of the seam, so that the liquid is retained, some solutions have already been developed, for example those described in patents US2010269245 or TW201004583. US2010269245, discloses a solution allowing a seam to be made by overlapping layers of a hot-melt material, this technique aiming at creating an impervious seam. However, this technique is only able to produce a sealing effect and not a retention effect.

TW201004583 discloses a solution allowing waterproofing effect on a zip fastener, which is applied onto clothing. This invention results from the application of two hot-melt tapes on each side of the zip fastener so as to ensure waterproofing of the fastener. However, despite the technique being similar to that of the present invention in terms of two tapes being placed on each side, the technique is only capable of waterproofing the zip fastener since the tapes are flat and cannot withstand liquid retention.

The already-cited WO 2011/108954, discloses a three-dimensional knitted fabric structure, but it is apparent that the same cannot totally retain the fluid as it leaks through the edges of the piece.

In this sense, a solution is now disclosed allowing the fluid to remain within the limits of the piece, thereby preventing leakage of fluids, in particular in undergarment items, while other advantageous features are obtained, including comfort and flexibility of the products, multifunctional characteristics thereof, resistance to washing and unsticking, as well as greater manufacturing ease.

### General description of the invention

A method is described for sealing absorbent structures with three dimensional knitted fabric structures and respective products.

A new method is thus described for sealing a three-dimensional knitted fabric used in male and female undergarments for urinary incontinence in order to prevent leakage of the liquid through the side edges of the absorbent centre area.

The advantage in allowing the sealing of the centre area of the piece, is due to the fact that the centre area consists, in various embodiments of the present technical field, of two pieces of fabric joined by a seam, this seam being responsible for leakage possibility. In other embodiments, it is possible to have more than two seams by connecting two or more pieces of fabric. In order to prevent such leakage, a method of applying a polyurethane-based tape has been developed, which provides essential advantages such as the adhesion ability, i.e. ability to "hot-glue" to the absorbent structure, a capacity allowing a molecular adhesion to the absorbent structure. This capability results in an increase in wash, wear and tear resistance. This bonding is especially interesting in the case of three-dimensional knitted fabric, in which adherence to the mesh structure itself is relevant.

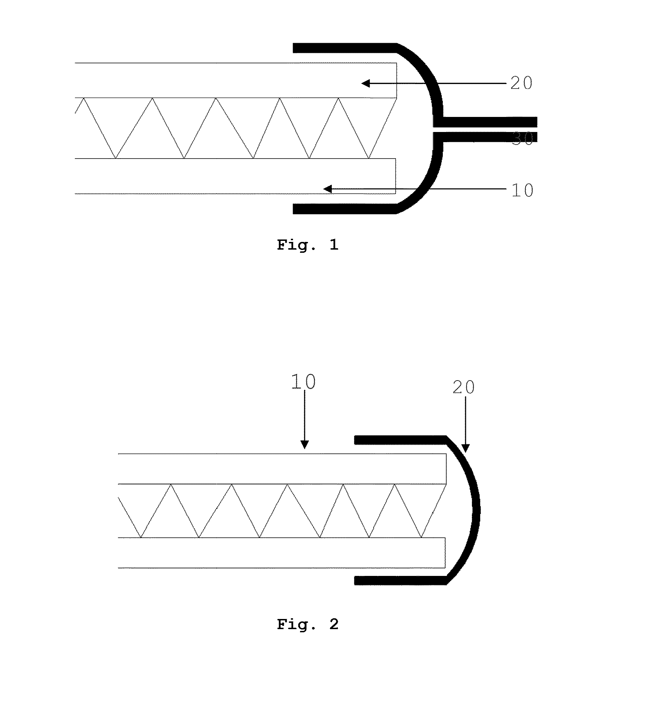

Particularly in the case of side sealing of the fabric, the tape consisting of polyurethane and polyester is applied around the entire perimeter of the absorbent structure by means of temperature and pressure exerted by the machine and with the aid of an adhesive placed onto the tape. The tape is inserted laterally into "U" shape and this "U" is provided by an auxiliary device developed for this purpose. Through its geometric configuration, the tape is inserted in flat form and acquires the "U" shape, since this shape allows liquid to be stored in the absorbent structure, thus creating a sort of enduring protection along the entire structure. As the example in figure 2.

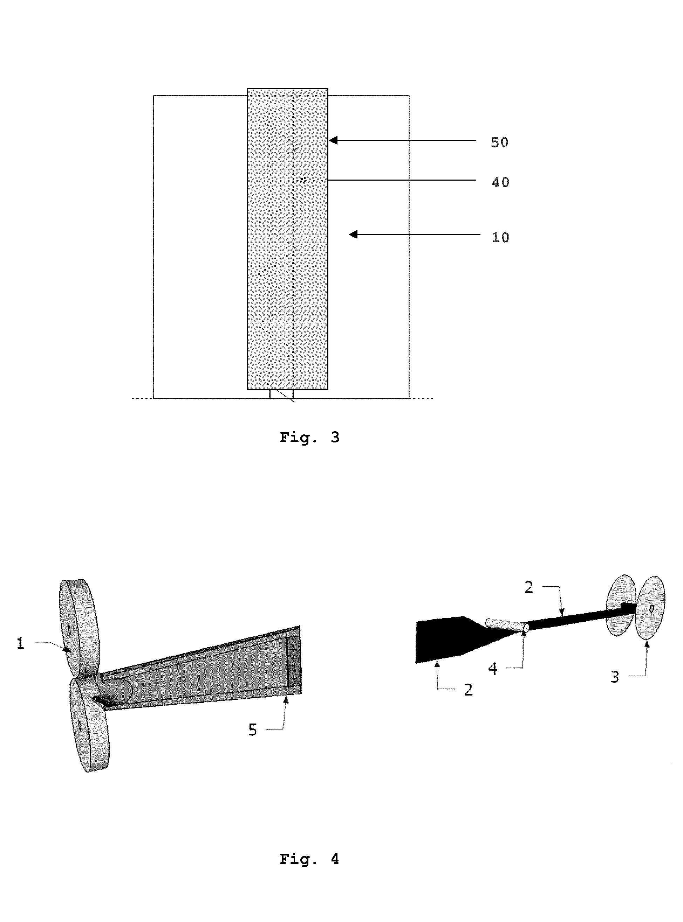

The application of the tape is carried out by adaptation made inside a hot seal taping machine, for example a MACPI machine, such as the MACPI 336 or 335, or any other machine capable of seal taping, available from any other manufacturer. This adaptation arose from the need to apply the tape in a U shape configuration, without creating defects and without compromising productivity. Thus, a guiding device for the tape has been developed, which is applied onto the physical structure of the machine, which undertakes tape insertion, thus facilitating the entry of the piece in the application process and, on the other hand, assuring uniform U-shape application of the tape, without compromising productivity. As the example in Figure 4.

The reference to "U" shape may also be understood as a "V" shape, the relevant aspect being folding the tape in order to follow-up both faces during the fabric edge sealing.

The selected tape is preferably composed of polyurethane and polyester, preferably having a weight between 150 and 500 g/m², more preferably between 250 and 390 g/m², in particular about 320 g/m², preferably a thickness between 0.20 and 0.70 mm, more preferably between 0.35 and 0.55 mm, particularly about 0.44 mm and a width preferably between 10 and 30 mm, more preferably between 17 and 28 mm, in particular about 22 mm. Ideal application conditions for the tape are: temperature: preferably between 210 and 270 °C, more preferably between 240 and 260 °C, more preferably between 245 and 255 °C, particularly about 250 °C; Application rate: preferably between 1 - 3 m/min, more preferably between 1 to 2.5 m/min, more preferably between 1.5 - 2 m/min, particularly about 1.5 m/min; and a Pressure preferably between 0.3 MPa - 0.45 MPa, more preferably between 0.35 - 0.4 MPa, in particular about 0.35 MPa.

The temperature preferably ranges between 210 and 270 °C, and the proper temperature to obtain an optimum fixation of the tape to the substrate is at an intermediate temperature. The application rate of the tape is determined by the time required for bonding, the preferred rate being 1 - 3 m/min. In turn, a pressure must occur during application of the tape, so that adhesion takes place without deformations or reliefs, said pressure being preferably in the range between 0.3 MPa - 0.45 MPa.

Especially in the case of centre sealing, a tape consisting of polyurethane was used, having been applied in the centre area on the centre seam, as shown by the example in Figure 3.

The selected tape consists preferably of polyurethane, has a weight preferably between 45 and 135 g/m², more preferably between 70 and 110 g/m², in particular about 90 g/m², having a thickness preferably between 0.04 and 0.16 mm, more preferably between 0.06 and 0.12 mm, particularly about 0.08 mm and a width preferably between 10 and 35 mm, more preferably between 17 to 28 mm, particularly about 22 mm. Ideal application conditions of the tape are: temperature: preferably between 210 and 270 °C, more preferably between 240 and 260 °C, more preferably between 245 and 255 °C, particularly about 250 °C; application rate: preferably between 1 - 4 m/min , more preferably between 2 - 3.5 m/min, more preferably between 2.5 - 3 m/min, particularly about 2.5 m/min; and a Pressure preferably between 0.3 MPa - 0.45 MPa, more preferably between 0.35 - 0.4 MPa, in particular about 0.35 MPa.

The temperature preferably ranges between 210 and 270 °C, and the proper temperature to obtain an optimum fixation of the tape to the substrate is at about 250 °C. The application rate of the tape is determined by the time required for bonding, the preferred rate being between 1 and 4 m/min. In turn, a pressure must occur during application of the tape, so that adhesion takes place without deformations or reliefs, said pressure being preferably in the range between 0.3 MPa - 0.45 MPa.

### Description of the Drawings

For an easier understanding, figures are herein attached, which represent preferred embodiments of the invention which, however, are not intended to limit the object of the present invention.
- Figure 1 -: Side Cut view, wherein (10) corresponds to the absorbent structure of the three-dimensional fabric; (20) corresponds to the "Y"-shaped tape for waterproofing and retention and (30) corresponds to the bonding area.
- Figure 2 -: Side cut view, wherein (1) corresponds to the absorbent structure; (20) corresponds to the "U"- or "V"-shaped tape for waterproofing and retention, and (30) corresponds to the bonding area.
- Figure 3 -: Scheme for the application of the tape, wherein (10) corresponds to the absorbent structure; (50) corresponds to the retention and waterproofing tape; (40) corresponds to the centre seam.
- Figure 4 -: Scheme of the equipment wherein (1) corresponds to pressure rollers; (2) corresponds to the waterproofing tape; (3) corresponds to the feeder; (4) corresponds the tension roller and (5) corresponds to the adapter of the tape.
- Figure 5 -: Scheme of the equipment wherein (1) corresponds to pressure rollers; (2) corresponds to the waterproofing tape; (3) corresponds to the feeder; (4) corresponds the tension roller; and (5) corresponds to the adapter of the tape.

### Detailed description of the invention

One embodiment includes two tasks comprising the side sealing of the edges of the absorbent structure and centre sealing of the same area. In general, embodiments should allow overcoming namely the following limitations: poor resistance to washing, with unsticking subsequently to a few number of washings, poor adhesion of both outer layers, and fluid leakage.

Several preferred embodiments shall be hereinafter described.

### Side sealing

In one embodiment, a tape consisting of polyurethane and polyester (superdolomite™) was used and was applied around the entire perimeter of the absorbent structure, and was glued to the outer and inner layers, with subsequent gluing of the layers so as to form a U-shaped side joining, by means of a fusible tape at a rate of about 1.4 m/min, at a pressure of about 0.35 MPa and at a temperature of about 270 °C.

Despite being possible for the uniformity of the gluing of the tape and the washing temperature to reach 90 °C, the operator(s) has(have) some difficulty in introducing the piece in the equipment.

In another embodiment, processability conditions were changed and a rate of 1.5 m/min and a temperature of about 250 °C were used.

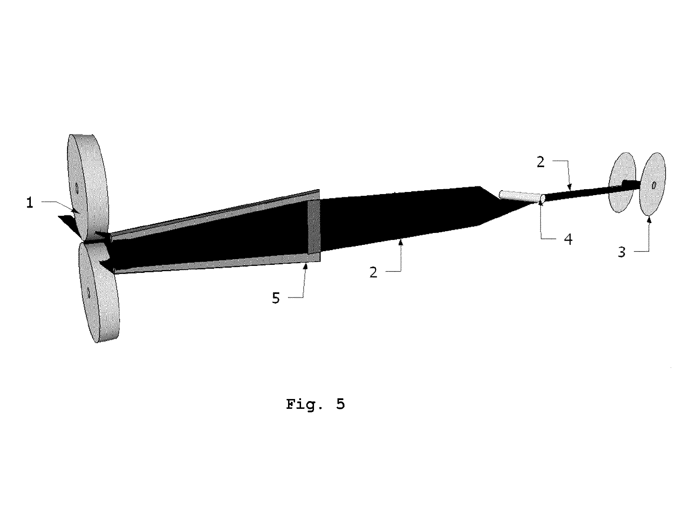

A special guiding device for the tape was also developed, which simplifies the insertion of the piece in the application process and ensures a uniform application and gluing of the tape (see Figures 4 and 5).

Thus, no problem related to resistance to washing, unsticking or leakage were registered.

Despite including a tape cover between the two faces of the fabric, by applying two tapes as shown in Fig. 1, "Y" sealing has provided inferior results, although being a possible embodiment.

### Centre sealing

In one embodiment, a tape was used consisting of nylon (Bemis™) and was manually applied onto the centre area, as shown in Figure 3. Application conditions were: temperature: 220 to 260 °C; application rate: 2.5 m/min; and pressure: 0.35 MPa.

This embodiment has poor resistance to washing, potential problems of centre leakage and may unstick at a temperature from 40 ° C.

In another embodiment, a tape was used consisting of polyurethane (Siena™) and was manually applied onto the centre area, as shown in Figure 3. Processability conditions were changed, and the temperature ranged between 220 and 240 °C, at an application rate of 2.5 m/min and at a pressure of 0.35 MPa.

Thus, no problem related to resistance to washing, unsticking or leakage were registered.

The embodiments described above may be combined together. The following claims define further preferred embodiments of the present invention.

## Claims

1. Method for sealing a three-dimensional knitted fabric (10) of an undergarment to retain body fluids, **comprising** the following steps:
a. application and hot-melt adhesion along the perimeter of the garment of a first tape (20), the tape (20) being face-to-face folded over the thickness of the garment;
b. application and hot-melt adhesion over and along the garment seams of a second tape (50);
wherein said tapes (20, 50) comprise hot-melt material.

2. Method according to the previous claim, **wherein** the hot-melt material is polyurethane.

3. Method according to the previous claims, **wherein** the first tape (20) comprises polyurethane and polyester, and has a weight between 150 to 500 g/m², a thickness between 0.20 and 0.70 mm and a width preferably between 10 and 30 mm.

4. Method according to the previous claims, **wherein** the second tape (50) comprises polyurethane, and has a weight between 45 and 135 g/m², a thickness between 0.04 and 0.16 mm and a width between 10 and 35 mm.

5. Method according to the previous claims, **wherein** the first tape (20) is applied and hot-melt adhered at a temperature between 210 and 270 °C, at an application rate of 1-3m/min and at a pressure of 0.3 MPa - 0.45 MPa.

6. Method according to claim 5, **wherein** the first tape (20) is applied and hot-melt adhered at a temperature between 240 and 260 °C.

7. Method according to the previous claim, **wherein** the first tape (20) is applied and hot-melt adhered at a temperature between 245 and 255 °C.

8. Method according to claim 5, **wherein** the first tape (20) is applied and hot-melt adhered at an application rate between 1 - 2.5 m/min.

9. Method according to the previous claim, **wherein** the first tape (20) is applied and hot-melt adhered at an application rate between 1.5 - 2 m/min.

10. Method according to claim 5, **wherein** the first tape (20) is applied and hot-melt adhered at a pressure between 0.35 Mpa - 0.4MPa.

11. Method according to the previous claims, **wherein** the second tape (50) is applied and hot-melt adhered at a temperature between 210 and 270 °C, at an application rate between 1 - 4 m/min and at a pressure between 0.3 Mpa - 0.45 MPa.

12. Method according to claim 11, **wherein** the second tape (50) is applied and hot-melt adhered at a temperature between 240 and 260 °C.

13. Method according to the previous claim, **wherein** the second tape (50) is applied and hot-melt adhered at a temperature of about 250 °C.

14. Method according to claim 11, **wherein** the second tape (50) is applied and hot-melt adhered at an application rate between 2 - 3.5 m/min.

15. Method according to the previous claim, **wherein** the second tape (50) is applied and hot-melt adhered at an application rate between 2.5 - 3 m/min.

16. Method according to claim 5, **wherein** the second tape (50) is applied and hot-melt adhered at a pressure between 0.35 MPa - 0.4 MPa.

17. Method according to the previous claims, **comprising** prior to the step of applying the first tape (20), the step of passing the tape (20) through a folder (5) so that it is provided with a longitudinal profile in a concave shape, so that tape (20) receives in such concavity the three-dimensional knitted fabric (10) of the garment.

18. Garment **wherein** it is obtained by any of the methods of the previous claims.

19. Garment according to the previous claim, **wherein** it is a male or female undergarment for urinary incontinence.

20. Machine for obtaining the sealing of a three-dimensional knitted fabric (10) of an undergarment to retain body fluids, **comprising:**
a. driving and pressure devices (1, 4) and temperature devices for hot-melting in a garment fabric of a tape (20, 50) comprising hot-melt material;
b. a feeder (3) for the tape (20);
c. a folder (5) for the tape (20) having longitudinal profile in concave shape;
wherein the folder (5) is arranged so as to receive the three-dimensional knitted fabric (10) of the garment, within the concavity of the concave longitudinal profile of the tape (20, 50).

21. Machine according to the previous claim, **comprising** means for hot-melt applying the tape (20) at a temperature between 210 and 270 °C, at an application rate of 1 - 3 m/min, and at a pressure of 0.3 MPa - 0.45 MPa.
